# EUROPEAN PATENT SPECIFICATION

(11) **EP 1 401 369 B1**
(45) Date of publication and mention of the grant of the patent: **13.09.2006**
(21) Application number: 02733719.5
(22) Date of filing: 17.05.2002
(51) Int. Cl.: A61F 13/64

(54) **ABSORBENT ARTICLE**
SAUGFÄHIGER ARTIKEL
ARTICLE ABSORBANT

(30) Priority: 21.05.2001 SE 0101822
(43) Date of publication of application: 31.03.2004
(73) Proprietor: SCA Hygiene Products AB, 405 03 Göteborg (SE)
(72) Inventor: KUSIBOJOSKA, Liljana, S-254 39 Helsingborg (SE); HERMANSSON, Kent, S-426 54 Västra Frölunda (SE)
(74) Representative: Egeröd, Lisbeth
(86) International application number: PCT/SE2002/000963
(87) International publication number: WO 2002/094165

(56) References cited:
- WO-A1-99/21522
- US-A- 5 135 522
- US-A- 5 695 849

## Description

### Technical field

The present invention relates to an absorbent article such as a diaper and incontinence guard comprising a liquid impervious topsheet, a liquid impervious backsheet and an absorbent body enclosed therebetween, whereby the article seen in longitudinal direction exhibits a front portion, a rear portion and a crotch portion therebetween, and further is provided with a pair of belt portions attached to the rear portion alternatively the front portion of the article and which are intended to be fastened together around the waist of the wearer by means of first fastening means and where said front portion alternatively the rear portion is provided with second fastening means intended to be attached to the belt portions, in such a way that the article will assume a pantlike shape, where the belt portions form a part of the waist portions of the pant.

### Background of the invention

Diapers and incontinence guards for incontinent adults usually have a garment portion holding an absorbent body in place against the user's body and attachment means, which hold the garment portion in place also when the user is moving. A common type of attachment means are adhesive tapes or hook and loop fasteners of the touch-and-close type which directly attach the front and rear portions of the absorbent article to each other. It is further known, through e.g., EP-A-0 287 388, EP-A-0 409 307, EP-A-0 528 282, EP-A-0 605 012 and FR-A-2 586 558, to attach the front and rear portions of the article by means of a belt, at which the possibilities to adjust the fit are improved. The belt further provides a simplified change of diaper or incontinence guard, especially when the patient is standing up.

On a common type of belt diaper the belt portions are first attached around the waist of the patient and then the front portion of the diaper is attached to the outside of the belt using fastening means being arranged on the front portion, whereby the outside of the belt serves as a reception surface for said fastening means.

Traditional belt materials have a tendency to fold themselves longitudinally upon usage and lie too close against the body. This is usually due to that the material does not exhibit the proper stiffness to be able to resist the different movements of the body and the pressure against the belt.

The stiffness is usually improved by using a thicker material or a material being strongly bonded. Using this approach, the breathability of the material is considerably deteriorated and the belt feels warm and tight against the skin.

### Summary of the invention

The object of the present invention is to provide a belt to a diaper or an incontinence guard being provided with a belt allowing an improved breathability. This object has been solved in that said belt portions exhibit a plurality of stiffening elements, being arranged on the side of the belt portions facing the wearet at a certain distance from each other, in such a way that air gaps are formed between the skin of the wearer and the side of the belt facing the wearet between said stiffening elements, and a largest extension of said stiffening elements is arranged across the belt in relation to the longitudinal direction of the belt. A possibility is thereby created for exchange of air between the skin and the belt. In addition, the stiffening elements contribute to an improved stability and stiffness in the transversal direction of the belt.

According to another embodiment of the invention, the stiffening elements exhibit an outer wall of a steam and air permeable material having the capability to transport steam and an inner core comprising a material having a higher steam and air permeability than the outer wall of the stiffening elements. This design makes it possible to transport moisture and steam away from the skin being under the belt and leads to the diaper feeling more comfortable to wear.

Further characteristics of the invention are evident from the following description and the dependent claims.

### Brief description of drawings

The invention will in the following be closer described with reference to an embodiment shown in the accompanying drawings.
Fig. 1 shows schematically a perspective view from above of a diaper or incontinence guard according to the invention.
Fig. 2 shows a top view of a belt according to the invention.
Fig. 3 shows an alternative placement of the stiffening element according to the invention.

### Detailed description of the invention

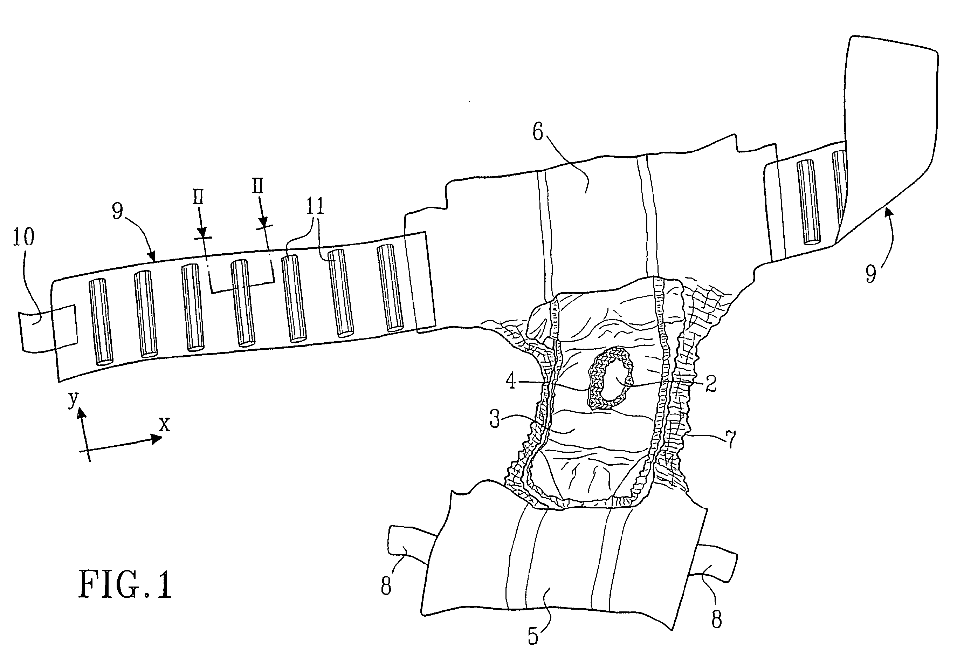

Fig.1 shows an embodiment of a diaper or incontinence guard 1 comprising a liquid impermeable backsheet 2, a liquid permeable topsheet 3 and an absorbent body 4 enclosed therebetween. The liquid permeable topsheet 3 can consist of a nonwoven material, e.g., a spunbond material of continuous filaments, a meltblown material, a bonded carded fibrous web or a perforated plastic film. The liquid impermeable backsheet 2 may consist of a plastic film, a nonwoven material coated with a liquid impervious material or a hydrophobic nonwoven material, which resists liquid penetration.

The backsheet material 2 and the topsheet 3 have a somewhat greater extension in the plane than the absorbent body 4 and extend outside the edges thereof The layers 2 and 3 are connected to each other within the projecting portions thereof, e.g., by gluing or welding using heat or ultrasound.

The absorbent body 4 can be of any conventional kind. Examples of commonly occurring absorbent materials are cellulosic fluff pulp, tissue layers, highly absorbent polymers (so called superabsorbents), absorbent foam materials, absorbent nonwovens or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well known to a person skilled in the art and does therefore not have to be described in detail. The thin absorbent bodies, which are common in for example baby diapers and incontinence guards often, comprise a compressed mixed or layered structure of cellulosic fluff pulp and superabsorbent.

The diaper/incontinence guard is intended to enclose the lower part of the wearer's trunk like a pair of absorbent pants. It exhibits a front portion 5 intended during use to be worn on the front part of the user's body, a rear portion 6 intended during use to be worn on the rear part of the user's body, and a crotch portion 7 located between the front and rear portions and which is intended to be worn in the crotch part of the user between the legs.

A pair of belt portions 9 is attached via one end, e.g., glued or ultrasonically welded, to the rear portion 6 of the diaper. The belt portions 9 are with their opposite ends intended to be fastened together, by means of first fastening means 10, which for example can be hooks and loop type of fasteners or adhesive tape tabs. The outside portions of the belt portions 9 are reception surfaces for the fastening means 10. The fastening means 8 of the front portion 5 are intended to be attached against the outside portions of the belt portions 9 in order to fasten together the diaper/incontinence guard to the desired pantlike shape. The fastening means 8 can be hooks and loop type of fasteners or adhesive tape tabs. According to an alternative embodiment, the belt portions 9 are attached to the front portion 5 of the diaper and thus are intended to be fastened together on the back of the wearer. The fastening means 8 are then arranged on the rear portion 6 of the diaper.

The width of the belt portions 9 should be between 5-20 cm, preferably between 7-15 cm. The belt portions 9 are preferably a laminate of a carrier material, which forms the outside of the belt, and a soft nonwoven, which forms the inside of the belt intended to be in direct contact with the skin of the user. A suitable nonwoven material can be a spunbond material of e.g., polypropylene- or polyethylene fibres. Conjugate fibres may also be used. Another suitable nonwoven material can be a carded thermobonded material of e.g., polypropylene, polyester or conjugate fibres. A nonwoven or another suitable material can be used as carrier material. The carrier material should be adapted to function as a reception surface for the fastening means 8 and 10. Elastic laminates are also suitable for use as material in the belt portions 9.

According to the invention, the belt portions 9 are provided with a plurality of stiffening elements 11, being arranged essentially across the longitudinal direction x of the belt. The wording "across" used here, means that they extend between the opposite longitudinal edges of the belt portions 9, either essentially perpendicular to these edges or obliquely in relation to these edges. However, they do not have to extend all the way to the longitudinal edges of the belt portions 9, but may stop slightly inside these edges. The stiffening elements 11 are arranged on the inside of the belt portions 12, i.e., the side facing the wearer. An air gap 16 is thereby formed between the skin of the user 15 and the inside of the belt 12 between the stiffening elements 11, leading to an increased exchange of air between the belt portions 9 and the skin 15. This leads to that the belt feels more comfortable for the user to wear since the belt in this way "breathes".

According to a preferred embodiment of the invention, the stiffening element 11 exhibits an outer wall and an inner core 14. The outer wall 13 of the stiffening element 11 comprises a steam- and air-permeable material and the inner core 14 comprises a material having a higher steam- and air-permeability than the outer wall 13 of the stiffening elements 11. Thereby a draught through said stiffening element 11 is achieved by a chimney effect, through which air and steam is transported away from the skin, up through the stiffening elements 11 and away from the belt.

Preferably, the material of the outer wall 13 of the stiffening elements 11 have a MVTR (Moisture Vapour Transmission Rate) value of at least about 2000 g/m³ /24 h or higher and the material of the inner core 14 a MVTR value exceeding the value for the outer wall 13 of the stiffening elements 11, leading to a transport of moisture taking place. The measuring method for estimation of MVTR is suitably ASTM E398, a method well known for a person skilled in the art.

The material of the outer wall 13 of the stiffening elements 11 is suitably a nonwoven material or a breathable plastic film. The inner core 14 preferably comprises an open foam material but may also consist of air. In the case of an air channel, a relatively high strength for the outer wall 13 of the stiffening element is required, in order to avoid that the channel collapses.

Upon usage of the stiffening elements 11 of the present invention, a belted article is achieved exhibiting a breathable belt, even if the material of the belt portions in itself is of a non-breathable material. The material in the belt portions can thus be freely chosen with respect to suitable properties such as strength, stiffness and density and still due to the stiffening elements 11 provide a breathable belt. It is also possible to chose a material, breathable or non-breathable, being well suited from a fastening point of view for the fastening means 10, but being too soft to alone function as a belt material, and which due to the stiffening elements 11 renders a higher stiffness. The stiffening elements 11 according to the invention are arranged on the inside 12 of the belt portions as being shown in Fig. 1, but the stiffening elements 11 could also be arranged within the belt portions 9, for example as a part of a laminate. The important thing is that the chimney-like shape and function of the stiffening elements 11 is maintained and that an air gap is formed between the skin 15 and the inside 12 of the belt portions.

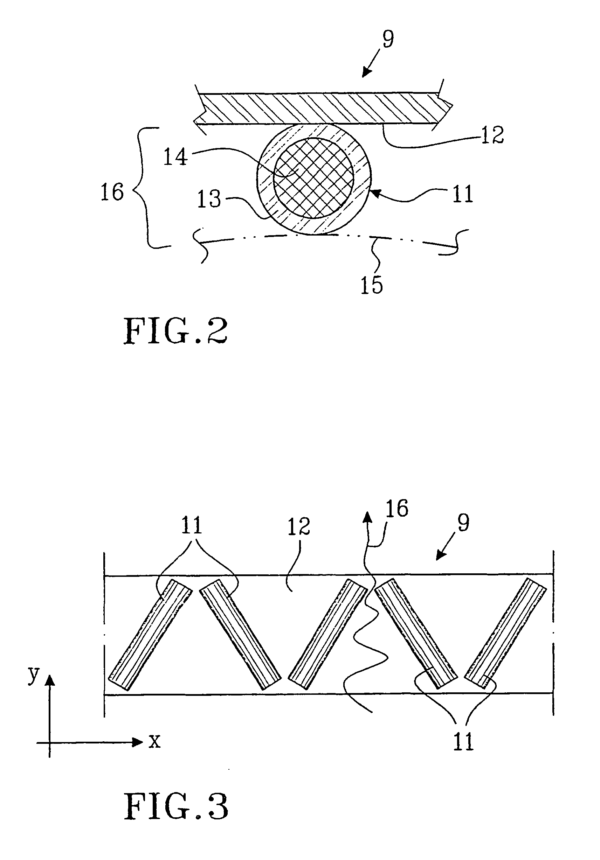

The distance between the stiffening elements 11 should preferably be between 1 and 7 cm. It is preferred that said stiffening element 11 are arranged across the longitudinal direction x as straight tube shaped element (see fig. 1), but said stiffening elements 11 can also be arranged in another pattern, for example in a zigzag pattern along the belt (see fig. 3), as long as the function of the stiffening elements is maintained. Also, the stiffening elements 11 can be arranged on some parts of the belt portions 9.

The invention is of course not limited to the above described and on the drawings shown embodiment, but can be modified within the scope of the claims.

## Claims

1. Absorbent article such as a diaper and incontinence guard comprising a liquid impervious topsheet (3), a liquid impervious backsheet (2) and an absorbent body (4) enclosed therebetween, whereby the article seen in longitudinal direction exhibits a front portion (5), a rear portion (6) and a crotch portion (7) therebetween, and further is provided with a pair of belt portions (9) attached to the rear portion (6) alternatively the front portion (5) of the article and which are intended by means of first fastening means (10) to be fastened together around the waist of the wearer and wherein said front portion (5) alternatively rear portion (6) exhibit second fastening means (8) intended to be attached to the belt portions (9), in such a way that the article will assume a pantlike shape, where the belt portions (9) form a part of the waist portions of the pant,
**characterised in,**
**that** said belt portions (9) exhibit a plurality of stiffening elements (11) arranged on the side (12) of the belt portions (9) facing the wearer at a certain distance from each other, in such a way that, when the article is worn, air gaps (16) can be formed between the skin of the wearer (15) and the side (12) of the belt facing the wearer between said stiffening elements (11), and a largest extension of said stiffening elements (11) is arranged essentially across the belt in relation to the longitudinal direction of the belt.

2. Absorbent article according to claim 1,
**characterised in,**
**that** said stiffening elements (11) comprise an outer wall (13) of a steam and air permeable material having the capability to transport steam and an inner core (14) comprising a material having a higher steam and air permeability than the outer wall (13) of the stiffening elements (11).

3. Absorbent article according to claim 2,
**characterised in,**
**that** the outer wall (13) of the stiffening element (11) exhibits a MVTR (Moisture Vapour Transmission Rate) value being at least 2000 g/m³ /24 h and that the inner core (14) of the stiffening elements (11) exhibits a MVTR value exceeding the MVTR value for the outer wall (13) of the stiffening elements (11).

4. Absorbent article according to any of the preceding claims,
**characterised in,**
**that** said stiffening elements (11) are arranged in a pattern, for example a striped pattern or a crosswise pattern.

5. Absorbent article according to any of the preceding claims,
**characterised in,**
**that** said stiffening elements (11) are arranged at a mutual distance between 1 cm and 7 cm.

## Patentansprüche

1. Absorbierender Gegenstand wie beispielsweise eine Windel und ein Inkontinenzschutz, umfassend eine flüssigkeitsdurchlässige Oberlage (3), eine flüssigkeitsundurchlässige Decklage (2) und einen dazwischen eingeschlossenen Absorptionskörper (4), wobei der Gegenstand gesehen in Längsrichtung einen Vorderabschnitt (5), einen Hinterabschnitt (6) und einen Schrittabschnitt (7) dazwischen aufweist und ferner mit einem Paar Gürtelabschnitte (9) versehen ist, die an dem Hinterabschnitt (6) alternativ dem Vorderabschnitt (5) des Gegenstandes angebracht sind und die dazu gedacht sind, mittels einer ersten Befestigungseinrichtung (10) um die Taille des Trägers aneinander befestigt zu werden und wobei der Vorderabschnitt (5) alternativ der Hinterabschnitt (6) eine zweite Befestigungseinrichtung (8) aufweist, die dazu gedacht ist, derart an den Gürtelabschnitten (9) angebracht zu werden, dass der Gegenstand eine höschenähnliche Form annehmen wird, wobei die Gürtelabschnitte (9) einen Teil des Taillenabschnitte des Höschens bilden,
**dadurch gekennzeichnet,**
**dass** die Gürtelabschnitte (9) mehrere Versteifungselemente (11) aufweisen, die in einem gewissen Abstand zueinander auf der Seite (12) der Gürtelabschnitte (9) angeordnet sind, die dem Träger zugewandt ist, so dass wenn der Gegenstand getragen wird, Luftspalte (16) zwischen den besagten Versteifungselementen (11) zwischen der Haut des Trägers (15) und der Seite (12) des Gürtels, der dem Träger zugewandt ist, gebildet werden können, und eine größte Dimension der Versteifungselemente (11) in Bezug auf die Längsrichtung des Gürtels im Wesentlichen quer zum Gürtel angeordnet ist.

2. Absorbierender Gegenstand nach Anspruch 1, **dadurch gekennzeichnet, dass** die Versteifungselemente (11) eine Außenwandung (13) aus einem dampf- und luftdurchlässigen Material mit der Fähigkeit, Dampf zu transportieren und einen Innenkern (14), umfassend ein Material mit einer höheren Dampf- und Luftdurchlässigkeit als die Außenwandung (13) des Versteifungselements (11) umfasst.

3. Absorbierender Gegenstand nach Anspruch 2, **dadurch gekennzeichnet, dass** die Außenwandung (13) des Versteifungselements (11) eine MVTR (Moisture Vapour Transmission Rate) von wenigstens 200 g/cm³/24 h aufweist und dass der Innenkern (14) des Versteifungselements (11) einen MVTR Wert aufweist, der den MVTR Wert der Außenwandung (13) der Versteifungselemente (11) überschreitet.

4. Absorbierender Gegenstand nach einem der vorstehenden Ansprüche, **dadurch gekennzeichnet, dass** die Versteifungselemente (11) in einem Muster, z.B. einem streifenförmigen Muster oder einem gekreuzten Muster, angeordnet sind.

5. Absorbierender Gegenstand nach einem der vorstehenden Ansprüche, **dadurch gekennzeichnet, dass** die Versteifungselemente (11) in einem gegenseitigen Abstand zwischen 1 cm und 7 cm angeordnet sind.

## Revendications

1. Article absorbant tel qu'une couche-culotte ou une protection contre l'incontinence comprenant une feuille antérieure (3) imperméable aux liquides, une feuille postérieure (2) imperméable aux liquides et un corps absorbant (4) enfermé entre elles, l'article présentant, vu dans la direction longitudinale, une partie avant (5), une partie arrière (6) et une partie entrejambe (7) entre elles, et est en outre pourvu d'une paire de parties de ceinture (9) attachées à la partie arrière (6) alternativement la partie avant (5) de l'article et qui sont destinées, au moyen de premiers moyens de fixation (10), à être attachées l'une à l'autre autour de la taille du porteur et dans lequel ladite partie avant (5) alternativement partie arrière (6) présente des deuxièmes moyens de fixation (8) destinés à être fixés aux parties de ceinture (9), de telle manière que l'article prend une forme de culotte, où les parties de ceinture (9) forment une partie des parties de taille de la culotte,
**caractérisé en ce que** lesdites parties de ceinture (9) présentent une pluralité d'éléments de raidissement (11) placés sur le côté (12) des parties de ceinture (9) tourné vers le porteur à une certaine distance les uns des autres, de telle manière que, lorsque l'article est porté, des coussins d'air (16) peuvent être formés entre la peau du porteur (15) et le côté (12) de la ceinture tourné vers le porteur entre lesdits éléments de raidissement (11), et la plus grande extension desdits éléments de raidissement (11) est disposée essentiellement en travers de la ceinture par rapport à la direction longitudinale de la ceinture.

2. Article absorbant selon la revendication 1, **caractérisé en ce que** lesdits éléments de raidissement (11) comprennent une paroi extérieure (13) d'un matériau perméable à la vapeur et à l'air ayant la capacité de transporter la vapeur et un noyau intérieur (14) comprenant un matériau ayant une plus grande perméabilité à la vapeur et à l'air que la paroi extérieure (13) des éléments de raidissement (11).

3. Article absorbant selon la revendication 2, **caractérisé en ce que** la paroi extérieure (13) des éléments de raidissement (11) présente une valeur de taux de transfert de la vapeur d'eau (MVTR) d'au moins 2 000 g/m³/24 h et **en ce que** le noyau intérieur (14) des éléments de raidissement (11) présente une valeur de MVTR supérieure à la valeur de MVTR de la paroi extérieure (13) des éléments de raidissement (11).

4. Article absorbant selon l'une quelconque des revendications précédentes, **caractérisé en ce que** lesdits éléments de raidissement (11) sont disposés en formant un motif, par exemple un motif à bandes ou un motif dans le sens transversal.

5. Article absorbant selon l'une quelconque des revendications précédentes, **caractérisé en ce que** lesdits éléments de raidissement (11) sont disposés à une distance mutuelle qui vaut de 1 cm à 7 cm.
